# EUROPÄISCHE PATENTSCHRIFT

(11) **EP 0 604 508 B1**
(45) Veröffentlichungstag und Bekanntmachung des Hinweises auf die Patenterteilung: **24.07.1996**
(21) Anmeldenummer: 92919593.1
(22) Anmeldetag: 18.09.1992
(51) Int. Cl.: A61F 2/30, A61F 2/38

(54) **ENDOPROTHESE MIT EINEM PROTHESENTEIL AUS VISKOELASTISCHEM KUNSTSTOFF**
ENDOPROSTHESIS WITH A VISCO-ELASTIC PLASTIC PROSTHESIS COMPONENT
ENDOPROTHESE COMPORTANT UNE PIECE PROTHETIQUE EN PLASTIQUE VISCO-ELASTIQUE

(30) Priorität: 19.09.1991 DE 9111729 U
(43) Veröffentlichungstag der Anmeldung: 06.07.1994
(73) Patentinhaber: Waldemar Link (GmbH & Co.), D-22315 Hamburg (DE)
(72) Erfinder: KELLER, Arnold, D-2061 Kayhude (DE)
(74) Vertreter: Glawe, Delfs, Moll & Partner
(86) Internationale Anmeldenummer: EP9202163
(87) Internationale Veröffentlichungsnummer: WO9305728

(56) Entgegenhaltungen:
- EP-A- 0 290 967
- WO-A-91/01691
- DE-A- 2 252 828
- US-A- 4 822 366

## Beschreibung

Die Erfindung betrifft eine Endoprothese gemäß den Merkmalen der gleichlautenden Oberbegriffe der Ansprüche 1 und 2, wie sie beispielsweise aus der US-A-4 822 366 bekannt ist.

Bei Endoprothesen muß häufig ein Teil aus viskoelastischem Kunststoff wie Polyethylen, das beispielsweise eine Gelenkfläche der Endoprothese bildet, an einem metallenen Trägerteil befestigt werden. Diese Schraube bedarf der Sicherung gegen ungewollte Drehung in Löserichtung. Im Maschinenbau ist eine Schraubensicherung bekannt (EP-A-0290967), die darauf beruht, daß die kraftübertragende Stirnfläche der Schraube mit radialen Rippen versehen ist, die sich in die Gegenfläche eingraben. Die Tiefe, bis zu der die Rippen in die Gegenfläche eindringen, wird bestimmt durch die Kraft, mit der die Schraube angezogen wird. Sie kann dadurch begrenzt werden, daß zwischen den Rippen vertiefte Anlageflächen vorgesehen sind, die sich bei der vorgesehenen Eindringtiefe der Rippen an die Gegenfläche anlegen. Jedoch ist die jeweilige Eindringtiefe der Rippen von der Schraubenkraft abhängig. Wenn die Schraube locker ist, wirkt auch die Sicherung nicht mehr. Hingegen soll in dem der Erfindung zugrundeliegenden Fall normalerweise keine oder nur geringfügige Schraubenkraft auf den zu befestigenden Kunststoffteil wirken; deshalb kann das bekannte Prinzip hier nicht direkt angewendet werden.

Die Erfindung sucht nach einer Schraubensicherung, die diesen speziellen Verhältnissen angepaßt ist. Die erfindungsgemäße Lösung liegt in den Merkmalen der Ansprüche 1 und 2.

Dadurch, daß die relative Lage der Sicherungsfläche des Schraubelements einerseits und der damit zusammenwirkenden Fläche des Kunststoffteils konstruktiv festgelegt werden, ist der Sicherungseingriff unabhängig von der Schraubenkraft. Auch ist dadurch Gewähr dafür gegeben, daß das Kunststoffteil nicht dadurch beschädigt werden kann, daß die Sicherungsfläche mit zu starker Montagekraft angezogen wird. Vielmehr wird unter allen Montagebedingungen dafür gesorgt, daß das Kunststoffmaterial nur in einem vorbestimmten Maß plastisch beansprucht werden kann.

Die Erhöhungen und Vertiefungen sind so gestaltet, daß lediglich eine plastische Materialverdrängung des Kunststoffs stattfindet, die später zumindest teilweise durch Relaxation wieder aufgehoben wird. Die Relaxation schafft eine gegenseitige Verzahnung der Sicherungsfläche mit der zugehörigen Kunststofffläche und damit den gewünschten gegenseitigen Sicherungseingriff zwischen Schraubelement und Kunststoff. Vorzugsweise sind die Erhöhungen bzw. Vertiefungen des Schraubelements in Umfangsrichtung sägezahnförmig gestaltet, wobei ihre steileren Zahnflanken entgegen der Löserichtung gerichtet sind.

Wenn zur Befestigung eine Schraube dient, kann diese an ihrem Ende ein Gewinde aufweisen, das mit einer Gewindebohrung des metallenen Trägerteils zusammenwirkt, und daran kopfseitig angrenzend einen verdickten Schaft, der einen Anschlag an dem metallenen Trägerteil bildet, so daß der Kopf sich in der Endlage der Schraube in einem vorbestimmten Abstand von der Oberfläche des metallenen Trägerteils befindet, der der Dicke des Polyethylenteils entspricht. Dabei wirkt der größte Teil der Schraubenkraft mit dem Trägerteil zusammen und nur ein kleiner Teil mit dem Polyethylenteil. Dadurch wird die Lage der Sicherungsfläche konstruktiv festgelegt. Diese Schraubenform ist an sich bekannt (US-A 48 22 366), aber nicht im Zusammenhang mit einer Schraubensicherung.

Bei einer anderen Ausführungsform der Erfindung findet die konstruktiv vorausbestimmte Festlegung der Sicherungsfläche dadurch statt, daß sie an einer Umfangsfläche angeordnet ist und mit einer Bohrung von entsprechendem Untermaß im Kunststoffteil zusammenwirkt.

Die Erfindung wird im folgenden näher unter Bezugnahme auf die Zeichnung erläutert, die Ausführungsbeispiele veranschaulicht. Es zeigen:
- Fig. 1: einen Längsschnitt durch eine erste,
- Fig. 2: einen Längsschnitt durch eine zweite Ausführungsform und
- Fig. 3: eine Draufsicht auf die zweite Ausführungsform.

Auf dem metallenen Trägerteil 1 mit Gewindebohrung 2 ist mittels der Schraube 3 das Polyethylenteil 4 zu befestigen. Da die beim Anziehen der Schraube üblicherweise auftretende Kraft größer ist als die Kraft, die man dem Polyethylen zumuten will, ist zwischen dem Gewinde 5 und dem Kopf 6 der Schraube ein Schaft 7 größeren Durchmessers angeordnet, der eine Anschlagfläche 8 bildet, die mit einer Anschlagfläche 9 an dem Trägerteil 1 zusammenwirkt, wenn die Schraube die vorgesehene Endlage erreicht hat.

Zur Aufnahme der Schraube weist der Polyethylenteil 4 eine Bohrung 10 auf, die zur Aufnahme des Schraubenkopfes 6 bei 11 erweitert ist. Die Unterfläche 12 des Schraubenkopfs wirkt daher zusammen mit der Spannfläche 13 des Polyethylenteils. Normalerweise würde man die Distanz zwischen der Unterfläche 12 und der Anschlagfläche 8 der Schraube etwa genauso groß machen wie die Distanz zwischen der Spannfläche 13 des Polyethylenteils und der Anschlagfläche 9 des Trägerteils. Wenn die Anschlagflächen 8 und 9 unter Spannung aneinanderliegen, um die Endlage der Schraube zu bestimmen, länge dann die Unterfläche 12 des Schraubenkopf 6 gerade auf der Spannfläche 13 des Polyethylenteils auf.

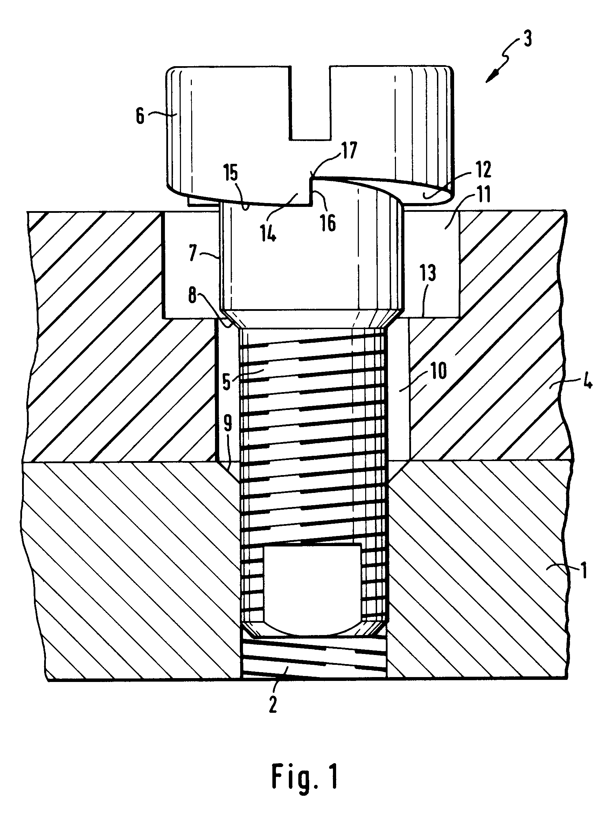

In der Ausführung gemäß Fig. 1 ist die Unterfläche 12 des Schraubenkopfes jedoch als Sicherungsfläche mit in Umfangsrichtung verlaufenden Sägezähnen 14 versehen, die einerseits von den flachen Flanken 15 und andererseits von den steilen Flanken 16 begrenzt werden, wobei diese Flächen so gelegen sind, daß die flachen Flanken 15 beim Anziehen der Schraube und die steilen Flanken 16 beim Lösen der Schraube vorangehen. Ferner ist der Abstand zwischen den Spitzen der Zähne 14 und der Anschlagfläche 8 geringer als der Abstand zwischen der Spannfläche 13 und der Anschlagfläche 9, so daß beim Anziehen der Schraube die Sägezähne 14 sich in das nachgiebige Polyethylenmaterial der Anschlagfläche 13 eingraben, wobei Material aus dem Weg der Sägezähne 14 zumindest teilweise plastisch verdrängt wird. Das hat zur Folge, daß unmittelbar nach dem Anziehen den steilen Flanken 16 kein oder nur wenig Kunststoffmaterial gegenübersteht, das das Lösen der Schraube verhindern könnte. Da aber Polyethylen und viele andere Kunststoffe, die daher ebenfalls für den Zusammenhang der Erfindung geeignet sind, viskoelastisch sind und daher Relaxation zeigen, d.h. die Eigenschaft, sich nach einer Verformung langzeitig zurückzuverformen in eine Gestalt, die der ursprünglichen Gestalt nahekommt, dringt allmählich wieder Kunststoff ein in die Leerstellen zwischen benachbarten Sägezähnen 14 und füllt damit auch den Raum vor den steilen Flanken 16 zumindest teilweise. Ein Lösen der Schraube kann daher nicht stattfinden, ohne daß dieses Kunststoffmaterial entfernt wird, was wegen der Steilheit dieser Flanken nur mit erheblichem Kraftaufwand möglich ist. Dieser Kraftaufwand ist um so größer, als die Verformung wegen der Flankensteilheit nicht durch einfache plastische Verdrängung stattfinden kann, sondern Zerstörung des Kunststoffmaterials voraussetzt. Dies reicht vollkommen aus, um Lösesicherheit gegenüber den geringen Kräften zu geben, die in den beschriebenen Einsatzfällen zu erwarten sind.

Die Höhendifferenz zwischen der Anschlagfläche 9 des Trägerteils 1 und der Spannfläche 13 des Polyethylenteils kann genauso groß sein wie die Höhendifferenz zwischen der Anschlagfläche 8 der Schraube und dem Zahngrund 17 der Sägezähne, so daß das volle Sägezahnvolumen für die Verdrängung des Kunststoffs zur Verfügung steht. Jedoch kann die Höhendifferenz zwischen den Flächen 9 und 13 auch etwas geringer sein, nämlich etwa dem mittleren Abstand zwischen der Anschlagfläche 8 und der flachen Flanke 15 der Sägezähne entsprechen. In einem solchen Fall entspricht das durch die vordringende Hälfte der Sägezähne verdrängte Materialvolumen etwa demjenigen Volumen, das in der oberen Hälfte der Zahnlücken zur Aufnahme des verdrängten Kunststoffmaterials zur Verfügung steht. Die optimale Bemessung kann leicht durch Versuch ermittelt werden.

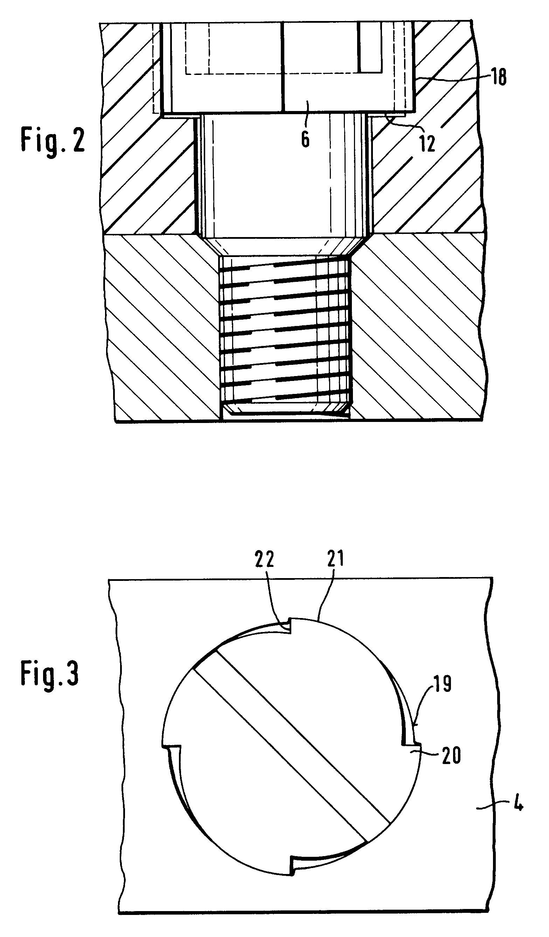

Die Anordnung gemäß Fig. 2 und 3 gleicht, soweit im folgenden nicht anders beschrieben, derjenigen nach Fig. 1. Der Unterschied besteht darin, daß nicht die Unterfläche 12 des Schraubenkopfs 6 zur Bildung der Sicherungsfläche benutzt wird, sondern seine Umfangsfläche 18, die mit der Oberfläche 19 der den Schraubenkopf 6 umgebenden Bohrung im Kunststoffteil 4 zusammenwirkt. Die Umfangsfläche 18 ist mit Sägezahnerhebungen 20 versehen, deren in Einschraubrichtung vorne liegende Flanke 21 sanft ansteigt, um eine plastische Verdrängung des umgebenden Materials zu ermöglichen, während die Rückseite 22 steil abfällt, um einer Drehung in Löserichtung zu widerstehen und das Kunststoffmaterial zu zerstören. Man erkennt in der Darstellung gemäß Fig. 3, daß die Oberfläche 19 des Kunststoffmaterials nach der plastischen Verdrängung während des Einschraubens der Schraube 3 in die zwischen den Sägezähnen 20 gebildeten Vertiefungen wieder eingeflossen ist und dadurch formschlüssig mit den Rückflanken 22 der Sägezähne zusammenwirkt.

Eine Sägezahnform ist zur Verfolgung der erfindungsgemäßen Zwecke notwendig. Jedoch sind auch solche sägezahnförmigen Vorsprünge auf der Unterfläche der Schraubelemente geeignet, die bei der Lösebewegung keine größere Kraftschwelle als beim Anziehen der Schraube verlangen, da zur Sicherung der Schraube sehr häufig auch geringere Verformungskräfte ausreichen.

Der Begriff Schraubelement schließt Schrauben, Muttern und darüber hinaus alle solche Elemente ein, die durch eine Schraubbewegung in Kontakt zu einer Spannfläche gebracht werden können.

Ein vorteilhafter Nebeneffekt der Erfindung besteht darin, daß durch die Zerstörung des Kunststoffs beim Lösen der Schraube die unzulässige Widerverwendung eines Kunststoff-Prothesenteils zuverlässig verhindert werden kann.

## Patentansprüche

1. Endoprothese mit einem an einem Trägerteil (1) der Prothese mittels eines Schraubelements (3) zu befestigenden Prothesenteil (4) aus viskoelastischem Kunststoff, insbesondere Polyethylen, wobei das Material des Trägerteils widerstandsfähiger ist als das des Kunststoff-Prothesenteils, dadurch gekennzeichnet, daß das Schraubelement (3) an seiner dem Kunststoff-Prothesenteil zugewendeten Stirnfläche (12) sägezahnförmige Erhöhungen (14) mit entgegen der Löserichtung gerichteten steileren Zahnflanken (16) aufweist, die in die Oberfläche (13) des Kunststoff-Prothesenteils derart eingreifen, daß eine viskoelastische Verdrängung des Kunststoffs stattfindet, wonach das verdrängte Kunststoffvolumen mindestens teilweise in die zwischen den Erhöhungen gebildeten Vertiefungen hinein relaxiert, wobei die Endlage des Schraubelements durch zusammenwirkende Anschlagflächen (8,9) an dem Schraubelement und dem Trägerteil (1) bestimmt ist.

2. Endoprothese mit einem an einem Trägerteil (1) der Prothese mittels eines Schraubelements (3) zu befestigenden Prothesenteil (4) aus viskoelastischem Kunststoff, insbesondere Polyethylen, wobei das Material des Trägerteils widerstandsfähiger ist als das des Kunststoff-Prothesenteils, dadurch gekennzeichnet, daß eine von einer Bohrungsfläche (19) des Kunststoff-Prothesenteils umgebene Umfangsfläche (18) des Schraubelements (3) sägezahnförmige Erhöhungen (20) mit entgegen der Löserichtung gerichteten steileren Zahnflanken (22) aufweist, die in die Oberfläche (19) des Kunststoffteils derart eingreifen, daß eine viskoelastische Verdrängung des Kunststoffs stattfindet, wonach das verdrängte Kunststoffvolumen mindestens teilweise in die zwischen den Erhöhungen gebildeten Vertiefungen hinein relaxiert, wobei die Bohrungsfläche (19) eine ursprüngliche Lage zwischen der Lage der Erhöhungen (20) und der Lage der Vertiefungen der Umfangsfläche (18) aufweist.

## Claims

1. An endoprosthesis with a prosthesis part (4) of viscoelastic plastics material, in particular polyethylene, to be fastened to a support part (1) of the prosthesis by means of a screw member (3), wherein the material of the support part is more resistant than that of the plastics prosthesis part, characterised in that on its end face (12) nearest the plastics prosthesis part the screw member (3) has sawtooth-like projections (14) with relatively steep tooth flanks (16) which are directed counter to the loosening direction and which engage into the surface (13) of the plastics prosthesis part in such a way as to induce a viscoelastic displacement of the plastics material, after which the displaced volume of plastics material relaxes at least partly into the recesses formed between the projections, the final position of the screw member being determined by co-operating abutment surfaces (8,9) on the screw member and on the support part (1).

2. An endoprosthesis according to Claim 1, with a prosthesis part (4) of viscoelastic plastics material, in particular polyethylene, to be fastened to a support part (1) of the prosthesis by means of a screw member (3), wherein the material of the support part is more resistant than that of the plastics prosthesis part, characterised in that a circumferential surface (18) of the screw member (3) surrounded by a bore surface (19) has sawtooth-like projections (20) with relatively steep tooth flanks (22) which are directed counter to the loosening direction and which engage into the surface (19) of the plastics prosthesis part in such a way as to induce a viscoelastic displacement of the plastics material, after which the displaced volume of plastics material relaxes at least partly into the recesses formed between the projections, the bore surface (19) having an original position between the position of the projections (20) and the position of the recesses in the circumferential surface (18).

## Revendications

1. Endoprothèse comportant une pièce prothétique (4) en plastique visco-élastique, en particulier en polyéthylène, destinée à être fixée à une pièce de support (1) de la prothèse au moyen d'un élément fileté (3), la matière de la pièce de support étant plus résistante que celle de la pièce prothétique en plastique, caractérisée en ce que l'élément fileté (3) présente, sur sa surface frontale (12) dirigée vers la pièce prothétique en plastique, des saillies en dents de scie (14) dont les flancs raides (16) des dents sont dirigés contre le sens de dévissage et qui s'engagent dans la surface (13) de la pièce prothétique en plastique de sorte qu'il se produise un refoulement visco-élastique du plastique, après quoi le volume refoulé de plastique se relâche au moins piècellement dans les creux formés entre les saillies, la position finale de l'élément fileté étant définie par des surfaces de butée coopérantes (8,9) sur l'élément fileté et la pièce de support (1).

2. Endoprothèse comportant une pièce prothétique (4) en plastique visco-élastique, en particulier en polyéthylène, destinée à être fixée à une pièce de support (1) de la prothèse au moyen d'un élément fileté (3), la matière de la pièce de support étant plus résistante que celle de la pièce prothétique en plastique, caractérisée en ce qu'une surface circonférentielle (18) de l'élément fileté (3), entourée par une surface de forure (19) de la pièce prothétique en plastique, présente des saillies en dents de scie (20) dont les flancs raides (22) des dents sont dirigés contre le sens de dévissage et qui s'engagent dans la surface (19) de la pièce en plastique de sorte qu'il se produise un refoulement visco-élastique du plastique, après quoi le volume refoulé de plastique se relâche au moins piècellement dans les creux formés entre les saillies, la surface de forure (19) présentant une situation primitive entre la situation des saillies (20) et la situation des creux de la surface circonférentielle (18).
